(19) Europäisches Patentamt / European Patent Office / Office européen des brevets

(11) **EP 3 367 968 B1**

(12) **FASCICULE DE BREVET EUROPÉEN**

(45) Date de publication et mention de la délivrance du brevet:
**28.02.2024 Bulletin 2024/09**

(21) Numéro de dépôt: **16787840.4**

(22) Date de dépôt: **26.10.2016**

(51) Classification Internationale des Brevets (IPC):
***A61F 2/06*** ***(2013.01)***

(52) Classification Coopérative des Brevets (CPC):
**A61F 2/06;** A61F 2002/061; A61F 2002/067;
A61F 2210/0014; A61F 2210/0057;
A61F 2220/0025; A61F 2230/0052;
A61F 2250/0098

(86) Numéro de dépôt international:
**PCT/EP2016/075786**

(87) Numéro de publication internationale:
**WO 2017/072168 (04.05.2017 Gazette 2017/18)**

(54) **COURONNE ÉLASTIQUE ET DISPOSITIF DE TRAITEMENT ASSOCIÉ POUR UNE IMPLANTATION DANS UN CONDUIT DE CIRCULATION D'UN FLUIDE CORPOREL**

**ELASTISCHER RING UND ZUGEHÖRIGE BEHANDLUNGSVORRICHTUNG ZUM IMPLANTIEREN IN EINE LEITUNG FÜR DIE ZIRKULATION EINER KÖRPERFLÜSSIGKEIT**

**ELASTIC RING AND ASSOCIATED TREATMENT DEVICE FOR IMPLANTING IN A CONDUIT FOR CIRCULATION OF A BODY FLUID**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.10.2015 FR 1560187**

(43) Date de publication de la demande:
**05.09.2018 Bulletin 2018/36**

(73) Titulaires:
- **ID Nest Medical**
  **67000 Strasbourg (FR)**
- **Université de Strasbourg**
  **67000 Strasbourg (FR)**

(72) Inventeurs:
- **CHAKFE, Nabil**
  **67150 Hindisheim (FR)**
- **CONTASSOT, David**
  **67100 Strasbourg (FR)**

(74) Mandataire: **Merckling, Norbert**
  **Cabinet Laurent et Charras**
  **1A Place Boecler**
  **CS 10063**
  **67024 Strasbourg Cedex (FR)**

(56) Documents cités:
EP-A1- 2 749 252    EP-A1- 2 749 252
EP-B1- 2 895 110    US-A1- 2003 120 292
US-A1- 2007 225 750    US-A1- 2007 225 750
US-A1- 2011 034 986    US-A1- 2011 034 986

**Description**

La présente invention concerne une couronne élastique définissant une ouverture intérieure, un contour externe et une surface d'appui annulaire entre l'ouverture intérieure et le contour externe, la couronne étant déformable entre une position de repos et une position d'introduction, dans laquelle l'ouverture intérieure présente un diamètre supérieur à son diamètre dans la position de repos, la couronne étant sollicitée élastiquement vers la position de repos.

L'invention concerne, comme décrit dans les revendications, un dispositif de traitement comprenant une telle couronne élastique disposée dans une fenêtre, sur le corps d'un implant. Un tel dispositif de traitement est destiné à être implanté dans une cavité d'un être vivant. La cavité est une région d'un réseau circulatoire d'un fluide corporel comportant au moins un conduit de circulation d'un fluide corporel, tel qu'un conduit de circulation du sang. En outre, le réseau comprend, par exemple, une branche faisant saillie transversalement à partir du premier conduit. Par exemple, dans la région de la cavité, le réseau présente des branches collatérales au conduit de circulation. Dans un exemple, le conduit de circulation du sang est l'aorte, dans laquelle est implanté un premier implant. Un deuxième implant est déployé dans une artère branchée sur l'aorte, telle que l'artère iliaque, l'artère rénale, l'artère mésentérique supérieure, le tronc coeliaque et les troncs artériels supra-aortiques, etc...

En variante, le premier implant est destiné à être disposé dans la crosse aortique. Le deuxième implant est alors placé dans une des branches débouchant dans la crosse aortique, telle que l'artère carotide primitive gauche, l'artère sous-clavière gauche ou le tronc artériel brachiocéphalique. D'autres applications sont possibles dans le système veineux, notamment dans la veine cave inférieure au niveau de la bifurcation iliaque, au niveau de l'abouchement des veines rénales et de la veine cave supérieure, au niveau de l'abouchement de ses branches collatérales.

Le document EP 2895110 décrit un dispositif de traitement comprenant un premier implant et un deuxième implant.
Un tel dispositif est généralement implanté dans un conduit de circulation du sang pour traiter des zones présentant des défauts ou des maladies, tels que des anévrismes des dissections, des lésions oblitérantes ou des compressions.

Pour implanter le dispositif, les techniques endoluminales sont actuellement préférées lorsqu'elles peuvent être mises en oeuvre. En effet, ces techniques sont moins invasives pour le patient et réduisent les taux de mortalité et de morbidité, ainsi que la durée de séjour à l'hôpital.

Les techniques endoluminales fournissent d'excellents résultats pour le traitement des lésions de l'aorte thoracique descendante, qui est linéaire. Cependant, dans le cas de la crosse aortique, l'implantation du dispositif par voie endoluminale présente un certain nombre de difficultés.

En premier lieu, le premier implant doit être suffisamment souple pour pouvoir s'adapter aux tortuosités de l'arbre artériel et se courber pour être amené de manière satisfaisante dans la crosse aortique puis déployé en se conformant à l'anatomie de ce vaisseau.

Ensuite, plusieurs implants secondaires doivent être montés transversalement à travers des fenêtres ménagées dans le premier implant. Ces implants secondaires ont pour rôle de fixer axialement le premier implant en évitant le risque de migration, et de couvrir les branches artérielles supra-aortiques afin d'assurer une revascularisation de ces branches.

Pour déployer un dispositif de traitement tel que précité dans un conduit de circulation du sang présentant une branche, il est connu tout d'abord de larguer le premier implant dans le conduit de circulation du sang. Une fenêtre ménagée dans le premier implant est disposée en regard de l'origine de chaque branche dans laquelle doit être implanté un deuxième implant. Puis, le deuxième implant est introduit dans la fenêtre du premier implant à travers la branche. Il existe deux sens d'introduction du deuxième implant dans la fenêtre du premier implant, lors de la pose du dispositif de traitement. Le deuxième implant est soit positionné dans la fenêtre du premier implant en venant de la lumière du premier implant, soit positionné dans la fenêtre du premier implant en allant vers la lumière du premier implant.

Le deuxième implant est ensuite déployé et est fixé sur le premier implant par l'intermédiaire d'un organe de retenue qui est par exemple une collerette déployable radialement à une extrémité du deuxième implant.

Pour assurer un bon fonctionnement du dispositif une fois implanté dans l'organisme, il est nécessaire de réaliser une étanchéité quasi-totale entre le contour intérieur de la fenêtre ménagée dans le premier implant et le contour extérieur du deuxième implant.

Cette étanchéité doit être maintenue tout au long de la présence de la prothèse dans le patient, notamment lorsque des déplacements du premier implant et/ou des implants secondaires se produisent.

Le document WO 2005/034810 décrit une prothèse comprenant une fenêtre et une couronne déformable du type précité, disposée autour de la fenêtre, pour maintenir un implant secondaire en position par rapport à un implant principal dans un dispositif de traitement. La déformabilité de la couronne autour de 4% limite l'effet du retour élastique de l'implant secondaire en acier après son implantation. Une telle couronne présente cependant une déformabilité très limitée. En particulier, le ressort hélicoïdal de forme annulaire assurant l'élasticité de la couronne est maintenu de manière très rigide dans un bord replié de l'ouverture, ce qui limite considérablement la déformabilité. L'étanchéité du dispositif n'est donc pas toujours parfaite, notamment si l'implant secondaire bouge, ou/et qu'il n'est pas aligné correctement avec un axe perpendiculaire à l'ouverture.

Cependant, pour assurer un bon fonctionnement du dispositif de traitement une fois implanté dans l'organisme, il est important de renforcer l'étanchéité d'un tel dispositif.

Un but de l'invention est de fournir une couronne élastique permettant d'assurer, de manière simple et robuste, une étanchéité très satisfaisante du dispositif de traitement durant toute la durée de son implantation dans le patient.

A cet effet, l'invention a pour objet un dispositif de traitement comprenant une couronne élastique du type précité, caractérisée en ce que la couronne comporte au moins trois brins traversant la couronne, chaque brin comportant une première extrémité et une deuxième extrémité positionnées sur le contour externe, le contour de l'ouverture intérieure étant défini par une portion de chaque brin et présentant une forme de pseudo-polygone.

La couronne élastique comprise dans un dispositif de traitement selon l'invention peut comprendre l'une ou plusieurs des caractéristiques suivantes, prise(s) isolément ou suivant toutes combinaisons techniquement possibles :

- les brins sont formés à partir d'un même fil, de préférence tressé ;
- les brins s'étendent sensiblement dans un même plan ;
- la largeur de la surface d'appui est comprise entre 30 % et 60 % du diamètre de l'ouverture intérieure dans la position de repos ;
- la couronne élastique comprend au moins une paire de brins comprenant deux brins appariés, les brins appariés étant des brins reliés entre eux à la fois par leur première extrémité et par leur deuxième extrémité ;
- les brins appariés définissent une forme oblongue, notamment une forme d'oeil ;
- les extrémités des paires de brins sont réparties sur le contour externe ;
- la couronne élastique comprend un nombre pair de brins, les brins étant appariés deux à deux, la couronne comprenant de préférence huit brins ;
- la déformation de la couronne est une déformation élastique permettant une augmentation réversible d'au moins 20 %, avantageusement d'au moins 30 % du diamètre de l'ouverture intérieure ;

traitement. La déformabilité de la couronne autour de 4% limite l'effet du retour élastique de l'implant secondaire en acier après son implantation. Une telle couronne présente cependant une déformabilité très limitée. En particulier, le ressort hélicoïdal de forme annulaire assurant l'élasticité de la couronne est maintenu de manière très rigide dans un bord replié de l'ouverture, ce qui limite considérablement la déformabilité. L'étanchéité du dispositif n'est donc pas toujours parfaite, notamment si l'implant secondaire bouge, ou/et qu'il n'est pas aligné correctement avec un axe perpendiculaire à l'ouverture.

Cependant, pour assurer un bon fonctionnement du dispositif de traitement une fois implanté dans l'organisme, il est important de renforcer l'étanchéité d'un tel dispositif.

Un but de l'invention est de fournir un dispositif de traitement comprenant une couronne élastique permettant d'assurer, de manière simple et robuste, une étanchéité très satisfaisante du dispositif de traitement durant toute la durée de son implantation dans le patient.

A cet effet, l'invention a pour objet un dispositif de traitement comprenant une couronne élastique du type précité, caractérisée en ce que la couronne comporte au moins trois brins traversant la couronne, chaque brin comportant une première extrémité et une deuxième extrémité positionnées sur le contour externe, le contour de l'ouverture intérieure étant défini par une portion de chaque brin et présentant une forme de pseudo-polygone.

Par le document EP 2,749,252 A1, on connaît une prothèse endoluminale constituée de brins formant une couronne. Chaque brin de ladite couronne comporte une extrémité positionnée sur le contour externe de la couronne et une autre extrémité positionnée sur le contour interne de la couronne, ce contour interne de la couronne étant de forme circulaire.

Par le document US 2003/0120292 A1, on connaît un dispositif d'anastomose prévu pour remplacer les sutures. Ce dispositif est formé de fils métalliques tressé en un cylindre. Il est destiné à être introduit au niveau d'une ouverture prévue sur deux tissus vivants en contact mutuel, puis à être déformé pour être aplati de manière à former deux évasements plats qui maintiennent les deux tissus vivants l'un contre l'autre par pincement desdits tissus entre les deux évasements plats.

La couronne élastique comprise dans le dispositif de traitement selon l'invention est décrite dans les revendications et peut comprendre l'une ou plusieurs des caractéristiques suivantes, prise(s) isolément ou suivant toutes combinaisons techniquement possibles :

- les brins sont formés à partir d'un même fil, de préférence tressé ;
- les brins s'étendent sensiblement dans un même plan ;
- la largeur de la surface d'appui est comprise entre 30 % et 60 % du diamètre de l'ouverture intérieure dans la position de repos ;
- la couronne élastique comprend au moins une paire de brins comprenant deux brins appariés, les brins appariés étant des brins reliés entre eux à la fois par leur première extrémité et par leur deuxième extrémité ;
- les brins appariés définissent une forme oblongue, notamment une forme d'oeil ;
- les extrémités des paires de brins sont réparties sur le contour externe ;
- la couronne élastique comprend un nombre pair de brins, les brins étant appariés deux à deux, la couronne comprenant de préférence huit brins ;

- la déformation de la couronne est une déformation élastique permettant une augmentation réversible d'au moins 20 %, avantageusement d'au moins 30 % du diamètre de l'ouverture intérieure ;
- chaque brin présente un diamètre compris entre 0,1 mm et 2 mm et de préférence de 0,2 mm.

L'invention a pour objet un dispositif de traitement comprenant :

- un premier implant comportant un corps, de préférence tubulaire, définissant une fenêtre,
- une couronne élastique telle que précédemment décrite, la couronne étant disposée sur le corps du premier implant dans la fenêtre, l'ouverture intérieure délimitant le passage d'introduction, d'un deuxième implant.

Le dispositif de traitement selon l'invention est décrit dans les revendications et peut comprendre l'une ou plusieurs des caractéristiques suivantes, prise(s) isolément ou suivant toutes combinaisons techniquement possibles :

- le corps du premier implant est tubulaire ;
- le corps est formé d'un treillis d'au moins un fil, la couronne étant formée sur le corps du premier implant dans la fenêtre à l'aide d'au moins un fil du treillis ;
- la couronne est rapportée sur le corps du premier implant dans la fenêtre ;
- la fenêtre est disposée sur la paroi du corps ;
- la fenêtre est disposée dans l'axe du corps.

L'invention sera mieux comprise à la lecture de la description qui va suivre, donnée uniquement à titre d'exemple, et faite en se référant aux dessins annexés, sur lesquels :

- la Figure 1 est une vue en perspective de trois-quarts face d'un premier dispositif de traitement selon l'invention, dans lequel un deuxième implant a été monté dans le premier implant ;
- la Figure 2 est une vue, prise en coupe suivant un plan transversal, du dispositif de la Figure 1 ;
- la Figure 3 est une vue de dessus de la couronne élastique dans une position de repos ;
- la Figure 4 est une vue de dessus de la couronne élastique dans une position d'introduction ;
- la Figure 5 est une courbe de la force produite par la couronne en fonction de l'augmentation de diamètre de l'ouverture intérieure de la couronne ;
- la Figure 6 est une vue schématique d'un ensemble de fabrication de la couronne élastique.

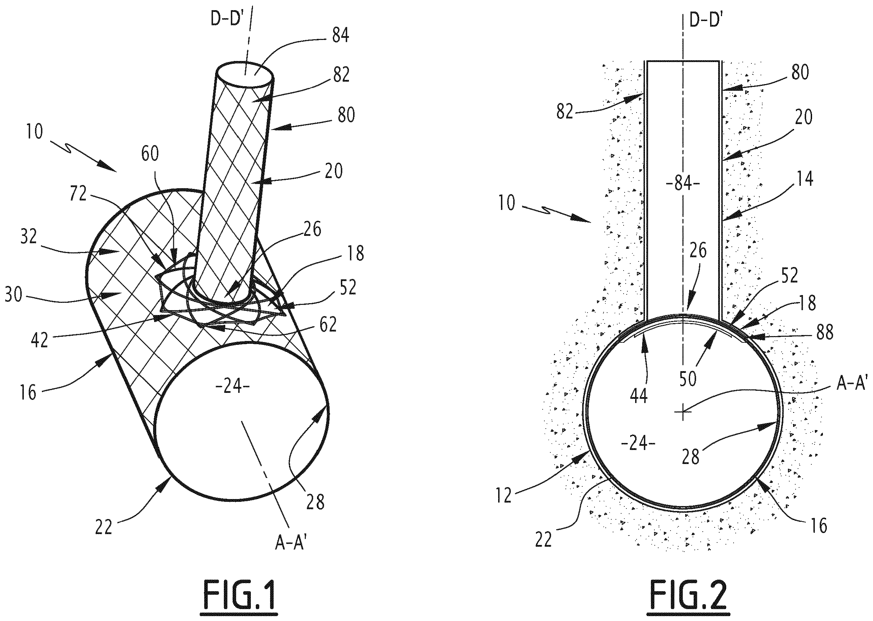

Un premier dispositif de traitement 10 selon l'invention est illustré par les Figures 1 à 4. Le premier dispositif de traitement 10 est destiné à être implanté dans une cavité intérieure définie dans le corps d'un être humain ou animal.

En référence à la Figure 2, la cavité comprend un tronçon principal 12 tubulaire et une branche 14 débouchant dans le tronçon principal 12.

Le tronçon principal 12 et la branche 14 sont, par exemple, des conduits de circulation du sang dans le système vasculaire d'un être humain ou animal. Le tronçon principal 12 est par exemple une artère ou une veine, et la branche 14 est un conduit de circulation du sang débouchant dans l'artère ou dans la veine.

En particulier, le tronçon 12 peut être l'aorte, notamment au niveau de la crosse aortique, ou un tronçon linéaire de l'aorte dans lequel sont connectés des segments d'endoprothèses abdominales.

Le premier dispositif de traitement 10 comporte un premier implant 16 et une couronne élastique 18.

En outre, tel qu'illustré sur les figures 1 et 2, le premier dispositif de traitement 10 comprend au moins un deuxième implant 20 destiné à être assemblé transversalement sur le premier implant 16 pour faire saillie à partir du premier implant 16 dans la branche 14. Dans le premier dispositif de traitement 10, la couronne élastique 18 permet de réaliser une étanchéité autour du deuxième implant 20.

Le premier implant 16 est destiné à être implanté dans le tronçon principal 12. Le premier implant 16 comporte un premier corps tubulaire 22 définissant un passage central 24 de circulation d'un fluide corporel et une fenêtre 26 débouchant dans le passage central 24.

Le corps tubulaire 22 présente une forme tubulaire autour d'un axe principal central A-A'. Il comporte une paroi périphérique 28 tubulaire formée avantageusement d'une armature ajourée 30 recouverte d'une couche 32 de revêtement.

Dans l'exemple le corps tubulaire présente une forme cylindrique. En variante, différentes sections du corps tubulaire 22 transversalement à l'axe central A-A' présente un diamètre différent. Par exemple, le corps tubulaire 22 présente une forme conique ou autre.

L'armature 30 est par exemple formée à partir d'au moins un fil élastique, réalisé par exemple en métal à mémoire de forme, tel qu'en Nitinol, ou en polymère. Le ou chaque fil est par exemple conformé en ziz-zag ou sous forme d'un treillis définissant des mailles.

La couche de revêtement 32 obture des ouvertures intermédiaires définies entre les tronçons de fil. Elle délimite de manière étanche le passage central 24 sur toute la longueur du corps 22, à l'exception de la fenêtre 26.

La couche de revêtement 32 est par exemple formée à base d'un film étanche aux liquides, présentant par exemple une épaisseur inférieure à 1 mm. Le film est par exemple réalisé en un polymère silicone, ou en un polymère fluoré tel que du PTFE. Dans un exemple, la couche de revêtement 32 est formée par un textile, par exemple tissé ou tricoté.

Le passage central 24 est délimité de manière étanche par la paroi périphérique 28. Il débouche axialement de part et d'autre du corps tubulaire 22 par une ouverture proximale et par une ouverture distale. Il débouche transversalement à travers la fenêtre 26.

La fenêtre 26 débouche à l'intérieur du passage central 24 et à l'extérieur du corps tubulaire 22.

Dans le premier dispositif de traitement 10, la fenêtre 26 est une fenêtre latérale. La fenêtre 26 est ménagée transversalement dans la paroi périphérique 28 à l'écart de l'ouverture proximale et de l'ouverture distale du corps tubulaire 22.

La fenêtre 26 permet l'insertion du deuxième implant 20.

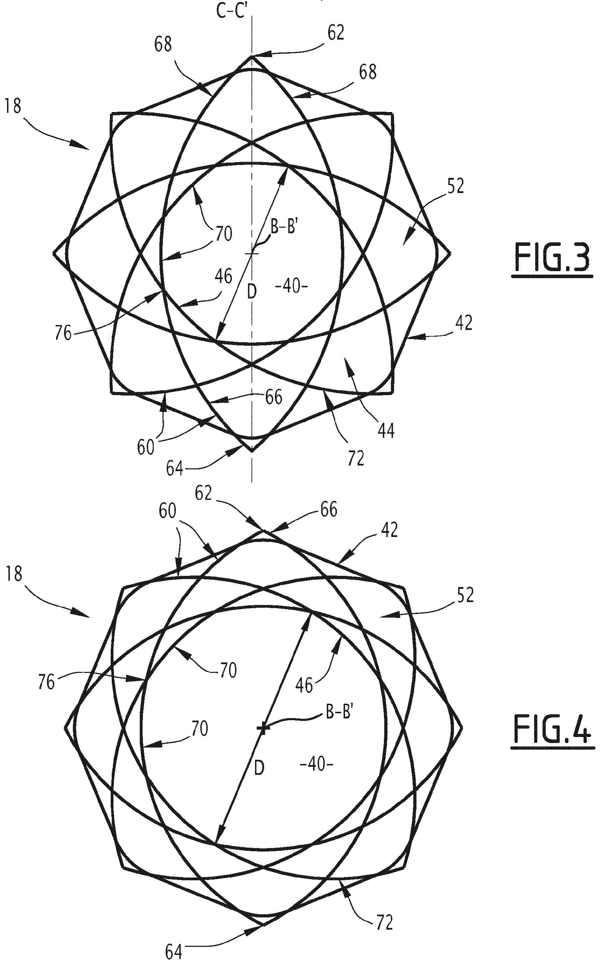

En référence aux figures 3 et 4, la couronne élastique 18, définit une ouverture intérieure 40, un contour externe 42 et une surface d'appui 44. L'ouverture intérieure 40 est délimitée par un contour 46. La surface d'appui 44 est définie entre le contour externe 42 et le contour 46 de l'ouverture intérieure 40. La surface d'appui 44 est annulaire.

La couronne élastique 18 est disposée sur le corps tubulaire 22 du premier implant 16 dans la fenêtre 26.

Dans le premier dispositif 10, la couronne 18 est rapportée sur le corps tubulaire 22 du premier implant 16 dans la fenêtre 22. Par exemple, la couronne 18 est fixée au corps tubulaire 22 autour de la fenêtre 26 par des organes de fixation (non représentés). Avantageusement, les organes de fixation sont disposés sur le contour externe 42 de la couronne 18.

La couronne 18 s'étend sensiblement circonférentiellement autour d'un axe central B-B'. Dans le premier dispositif 10, l'axe central B-B' de la couronne élastique 18 est sensiblement perpendiculaire à l'axe principal A-A' du premier implant 16. Par sensiblement perpendiculaire, il est entendu que les axes forment entre eux un angle compris entre 85 ° et 95°.

La couronne 18 présente une face intérieure 50 destinée à être en regard du passage central 24 et une face extérieure 52 destinée à être en regard de l'extérieur du premier implant 16. La couronne 18 est sensiblement plate.

La surface d'appui 44 de la couronne 18 présente une forme légèrement incurvée, sensiblement complémentaire de la paroi périphérique 28 du corps tubulaire 22 autour de la fenêtre 26.

La couronne élastique 18 comprend une couche de revêtement couvrant la surface d'appui 44 mais n'obturant pas l'ouverture intérieure 40. La couche de revêtement assure l'étanchéité de la surface d'appui 44 entre la face intérieure 50 et la face extérieure 52. La couche de revêtement est par exemple disposée sur la face extérieure 52 de la couronne élastique 18. En variante, la couche de revêtement est disposée sur la face intérieure 50 de la couronne élastique 18.

La couronne élastique 18 est avantageusement radio-opaque. Ceci permet de vérifier l'orientation de la fenêtre 26 dans laquelle est disposée la couronne élastique 18 par rapport à l'axe du tronçon principale 12 et de faciliter le placement en face de la branche 14.

Le contour externe 42 est formé d'au moins un tronçon de fil, ici une succession de tronçon de fils. Le contour externe 42 présente la forme d'un polygone régulier, inscrit dans un cercle, l'axe central B-B' passant par le centre du cercle. Lorsque la couronne 18 est dans le dispositif de traitement 10, la forme du contour externe 42 est par exemple maintenue par les points de fixation au premier implant 16.

Le diamètre du contour externe 42 est défini comme la distance maximale entre deux points du contour externe 42.

Le diamètre D de l'ouverture intérieure 40 est défini comme la distance maximale entre deux points du contour 46 de l'ouverture intérieure 40.

La couronne 18 est déformable entre une position de repos et une position d'introduction, dans laquelle l'ouverture intérieure 40 présente un diamètre supérieur à son diamètre dans la position de repos. La couronne 18 est représentée en position de repos sur la figure 3 et en position d'introduction sur la figure 4.

La couronne 18 est sollicitée élastiquement vers la position de repos.

La déformation de la couronne élastique 18 est une déformation élastique permettant une augmentation réversible d'au moins 20 %, avantageusement d'au moins 30 % du diamètre de l'ouverture intérieure 40.

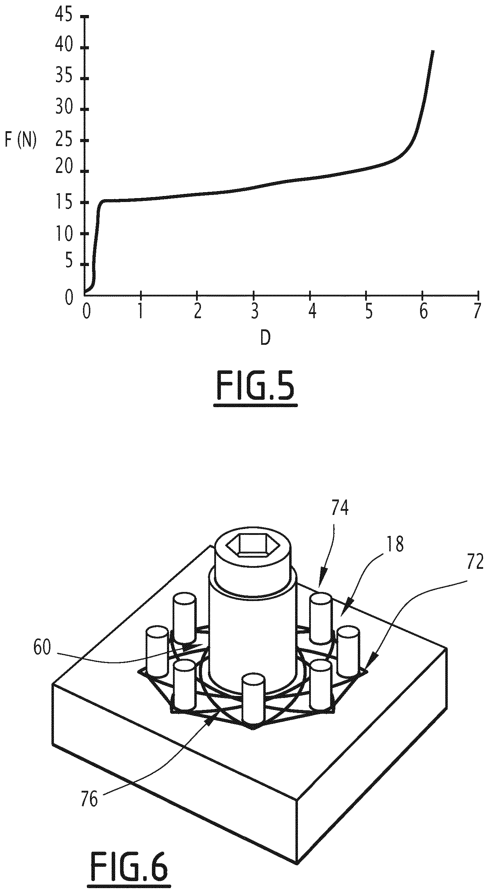

Lors de l'exercice d'une force d'écartement sur l'ouverture intérieure 40, la couronne élastique 18 exerce une force de résistance F opposée visible sur la figure 5. Plus le diamètre D de l'ouverture intérieure 40 est grand par rapport au diamètre de l'ouverture intérieure 40 en position de repos, plus la force de résistance est importante. La force de résistance est compensée par une réduction du diamètre du contour externe 42, tel que décrit par la suite. En effet, le diamètre du contour externe 42 diminue lorsque la force de résistance augmente.

La déformation réversible de la couronne élastique 18 résulte notamment de la présence de brins 60 propres à s'écarter.

La couronne élastique 18 comporte au moins trois brins 60 traversant la couronne élastique 18.

Chaque brin 60 comporte une première extrémité 62 et une deuxième extrémité 64 positionnées sur le contour externe 42. Par exemple, les extrémités 62, 64 sont engagées sur le contour externe 42.

Avantageusement, la couronne 18 comprend au moins une paire 66 de brins 60 comprenant deux brins appariés 68. Les brins appariés 68 sont des brins 60 ayant des extrémités communes, notamment leur première extrémité 62 et leur deuxième extrémité 64.

Avantageusement, la couronne 18 comprend un nombre pair de brins 60, les brins 60 étant appariés deux à deux.

Dans l'exemple représenté sur les figures 1 à 4, la couronne élastique 18 comprend huit brins 60 appariés deux à deux. Ainsi, la couronne élastique 18 de l'exemple comprend 4 paires 66 de brins appariés 68.

Chaque brin 60 est allongé entre la première extrémité 62 et la deuxième extrémité 64.

Chaque brin 60 présente un diamètre compris entre 0,1 mm et 2 mm, par exemple, de 0,2 mm.

Dans l'exemple représenté, les brins 60 ont tous la même longueur.

Le brin 60 est formé d'un fil élastique. Par exemple, le fil est réalisé en métal à mémoire de forme, tel qu'en Nitinol. En variante, le fil est réalisé en polymère.

Chaque brin 60 présente un module élastique, par exemple, compris entre 20 GPa et 200 GPa. L'élasticité du brin 60 contraint le brin 60 vers une configuration tendue dans laquelle le brin 60 est faiblement incurvé entre la première extrémité 62 et la deuxième extrémité 64.

Les seules intersections entre deux brins 68 d'une paire 66 sont à leurs extrémités 62, 64. La paire 66 de brins 68 définit un axe C-C' passant par la première extrémité 62 et la deuxième extrémité 64 des brins appariés 68.

Dans l'exemple, les extrémités 62, 64 du brin 60 sont retenues par le contour extérieur 42, chaque brin 60 présente donc un fléchissement entre ses extrémités 62, 64. Chaque brin 60 présente ainsi une forme incurvée.

Ainsi, une paire 66 de brin 68 présente une forme oblongue, notamment une forme d'oeil. On entend par forme d'oeil que chaque brin apparié 68 présente sensiblement la forme d'un arc de cercle, les courbures des arcs de cercle étant opposées.

La forme oblongue est, avantageusement, symétrique par rapport à l'axe C-C' de la paire 66 de brins 68.

Les brins 60 traversent la couronne 18. Cela signifie que l'angle entre la position angulaire de la première extrémité 62 et la position angulaire de la deuxième extrémité 64 d'un brin 60, par rapport à l'axe central B-B' de la couronne 18, est égal à 180°.

Avantageusement, la première extrémité 62 et la deuxième extrémité 64 de chaque brin 60 sont sensiblement diamétralement opposées par rapport à l'axe central B-B'.

L'axe C-C' de la paire 66 de brins 68 passe avantageusement par l'axe central B-B' de la couronne 18.

Les extrémités des paires 66 de brins 60 sont réparties angulairement sur le contour externe 42. Ceci permet de répartir tous les efforts autour du contour externe 42.

Les brins 60 s'étendent sensiblement dans un même plan.

L'ouverture intérieure 40 est un passage central débouchant dans la face intérieure 50 et dans la face extérieure 52 de la couronne 18.

Comme décrit précédemment, l'ouverture intérieure 40 présente un diamètre variable.

Lorsque la couronne est dans la position d'introduction, le diamètre de l'ouverture intérieure 40 est supérieur ou égal au diamètre du deuxième implant 20 pour permettre son insertion. Par exemple, le diamètre de l'ouverture intérieure 40 dans la position d'introduction est supérieur à 4 mm et avantageusement compris entre 4 mm et 10 mm.

Dans la position de repos, le diamètre de l'ouverture intérieure 40 est strictement inférieur au diamètre du deuxième implant 20. Par exemple, le diamètre de l'ouverture intérieure 40 dans la position d'introduction est inférieur à 10 mm et avantageusement compris entre 4 mm et 8 mm.

La largeur de la surface d'appui 44 est la différence entre le diamètre du contour externe 42 et le diamètre de l'ouverture intérieure 40. La largeur de la surface d'appui 44 est de préférence comprise entre 30 % et 60 % du diamètre de l'ouverture intérieure 40 dans la position de repos.

Le contour 46 de l'ouverture intérieure 40 est propre à se conformer au contour externe 42 du deuxième implant 20.

Le contour 46 de l'ouverture intérieure 40 est défini par une portion 70 de chaque brin 60. Le contour 46 de l'ouverture intérieure 40 présente une forme de pseudo-polygone. On entend par « pseudo-polygone », une figure fermée sensiblement plane délimitée par plusieurs segments reliés par des sommets. Par exemple, les segments sont sensiblement incurvés entre les sommets du pseudo-polygone.

Les sommets du pseudo-polygone sont situés à des intersections entre deux brins 60. En particulier les sommets sont situés à des intersections entre des brins 60 n'appartenant pas à la même paire 66.

Les sommets du pseudo-polygone sont disposés régulièrement autour de l'axe central B-B'. Ainsi, les sommets sont inscrits dans un cercle de centre passant par l'axe B-B'.

Avantageusement les sommets du pseudo-polygone sont sensiblement dans le même plan.

De plus, les intersections entre les brins 60 qui sont situées entre le contour externe 42 et l'ouverture intérieure 40 définissent la surface d'appui 44, sur laquelle le deuxième implant 20 repose une fois implanté dans la fenêtre 26. Cette surface d'appui 44 présente une aire supérieure à 80 % de l'aire de l'ouverture intérieure.

Avantageusement, les brins 60 sont formés à partir d'un même fil 72. Avantageusement, le fil 72 est également utilisé pour former le contour externe 42.

Le fil 72 est de préférence tressé, pour former des jonctions entre les brins 60. Les jonctions permettent de répartir les contraintes entre les brins 60.

La figure 6, présente un ensemble de fabrication de la couronne élastique permettant le tressage du fil 72.

Lors de ce tressage, le fil 72 est mis en place en suivant un chemin entre des plots 74 permettant de positionner les différentes intersections entre les brins 60 du fil 72.

A chaque intersection, le fil 72 passe en dessous ou au-dessus du brin de fil 72 croisé. Le tressage consiste en une alternance entre un passage du fil 72 en dessous du brin de fil 72 croisé et un passage du fil 72 au-dessus du brin 60 de fil 72 croisé entre deux intersections 76 successives, suivi par l'élaboration d'un tronçon du contour externe 42 pour atteindre une autre extrémité de deux brins 60 appariés.

La couronne élastique 18 ainsi formée présente donc une épaisseur maximale sensiblement égale à deux fois le diamètre du fil 72, en raison des intersections 76 où plusieurs brins 60 se chevauchent.

Par exemple, lorsque la couronne 18 est formée à partir d'un tressage, les intersections 76 permettent de définir les extrémités de chaque brin 60 sur le contour externe 42 et les sommets du pseudo-polygone.

La couronne 18 est ainsi une tresse à plat présentant une ouverture intérieure 40 extensible.

La nature du fil 72 des brins 60 permet un maintien de la mise en forme de la couronne optimale. Par exemple, pour l'implantation du dispositif, la couronne 18 est repliée dans un dispositif de largage du premier implant 16 et récupère après déploiement sa forme initiale optimale.

Comme illustré par les Figures 1 et 2, le deuxième implant 20 comprend un deuxième corps tubulaire 80 autour d'un axe principal central D-D'. L'axe principal central D-D' est sensiblement parallèle à l'axe central B-B' de la couronne 18.

Le deuxième corps tubulaire 80 comporte une paroi périphérique 82 définissant un passage central auxiliaire 84. Lorsque le deuxième implant 20 est inséré dans l'ouverture centrale 40, le passage central auxiliaire 84 débouche dans le passage central 24 du premier implant 16.

Comme pour la paroi périphérique 28 du corps tubulaire 22, la paroi 82 du deuxième corps 80 comporte une armature ajourée recouverte d'une couche de revêtement. Par exemple, la couche de revêtement du deuxième corps tubulaire 80 est dans le même matériau que la couche de revêtement 32 du premiers corps 22. L'armature est par exemple formée par au moins un fil conformé en zig-zag, ou par un treillis de fils définissant des mailles.

Comme illustré sur la figure 2, le deuxième implant 20 comporte une butée 88. Par exemple, la butée 88 comprend une collerette faisant saillie radialement par rapport à l'axe principal central D-D' du deuxième corps tubulaire 80, avantageusement perpendiculairement par rapport à l'axe principal central D-D'. La collerette est par exemple continue sur toute la périphérie du bord distal autour de l'axe principal central D-D'. En variante, la collerette est formée par une pluralité de doigts disjoints discontinus. Une fois le deuxième implant 20 monté sur le premier implant 16, la butée 88 est appliquée contre la surface d'appui 44 annulaire et éventuellement contre une surface intérieure du premier corps tubulaire 22 située autour de la fenêtre 26 pour empêcher le déplacement radial vers l'extérieur du deuxième implant 20 par rapport au premier implant 16. Le deuxième implant 20 présente ainsi une forme générale de T et est désigné par le terme « T-stent ».

La surface d'appui 44 annulaire étant large et bidimensionnelle, elle offre un appui robuste à la butée 88.

Le deuxième implant 20 est déformable entre une configuration rétractée d'introduction et une configuration déployée implantée dans la branche 14 et dans la fenêtre 26 du premier implant 16, telle que représentée sur la Figure 2. Avantageusement, le deuxième implant 20 est autoexpansible.

Dans la configuration rétractée, le deuxième implant 20 présente une étendue radiale minimale. Dans cette configuration, la butée est contractée radialement au voisinage de l'axe D'-D'. Le deuxième implant 20 est apte à être convoyée à travers le passage central 24 et la fenêtre 26 et à être introduit dans l'ouverture intérieure 40 de la couronne élastique 18. Dans la configuration déployée représentée sur la figure 2, le deuxième corps tubulaire présente une étendue radiale maximale autour de l'axe D-D'. La butée 88 est déployé transversalement par rapport à l'axe D-D'.

Dans un exemple, le deuxième implant 20 maintenu dans un lanceur dans la configuration rétractée, lorsque la couronne 18 est dans la position d'introduction, le diamètre de l'ouverture intérieure 40 est supérieur ou égal au diamètre du lanceur du deuxième implant 20.

Pour garantir une bonne étanchéité, le contour extérieur du deuxième implant 20 dans sa configuration déployée présente une étendue supérieure à l'étendue de l'ouverture intérieure 40 de la couronne 18 en position de repos. Le deuxième implant 20 déployé présente dans sa configuration déployé un diamètre extérieur supérieur au diamètre de l'ouverture intérieure 40 de la couronne 18 en position de repos.

Lorsque le deuxième implant 20 est inséré dans l'ouverture intérieure 40, la couronne 18 est déformée élastiquement autour du deuxième corps tubulaire 80. Le contour 46 de l'ouverture intérieure 40 présente alors une forme conjuguée au contour extérieur du deuxième corps 80 au contact de la couronne élastique 18.

Lorsque le deuxième implant 20 est reçu dans l'ouverture intérieure 40, une force radiale de retenue et d'étanchéité s'applique contre la surface extérieure du deuxième corps tubulaire 80, quelle que soit la conformation spatiale du deuxième corps tubulaire 80 par rapport au premier corps tubulaire 22. En particulier, l'étanchéité est réalisée sur tout le contour 46 de l'ouverture intérieure 40, par chacune des portions 70 de brin 60 délimitant le pseudo-polygone central.

La couronne élastique 18 garantit donc une étanchéité engendrant une force significative à l'interface entre le premier implant 16 et le deuxième implant 20 dans la fenêtre 26. En outre, la répartition des portions 70 de brins 60 est adaptée pour que la couronne 18 présente une force uniforme sur le deuxième implant 20, tout le long du contour 46 de l'ouverture intérieure 40.

Par ailleurs, le deuxième implant 20 est retenu de manière robuste dans le premier implant 16. La force nécessaire à l'arrachement du deuxième implant 20 hors de la couronne élastique 18 est par exemple supérieure à 10 N.

En particulier l'élasticité de la couronne 18 permet une adaptation de l'ouverture intérieure 40 en cas de rétractation du diamètre du deuxième implant 20 (appelé recoil en anglais). Le dispositif de traitement permet donc le maintien d'une pression continue et active sur le deuxième implant dans la branche 14.

Le dispositif 10 est propre à s'adapter aux mouvements et aux déformations relatifs entre le premier implant 16 et le deuxième implant 20, par exemple imposées par le flux systolo-diastolique de l'artère 12. En effet, le dispositif 10 est mobile lors du flux systolo-diastolique alors que la du deuxième implant dans la branche 14 collatérale est fixe. Il existe ainsi une adaptation permanente de l'étanchéité lors de ces micro-déplacements entre le deuxième implant 20 et la couronne 18 qui contraint en permanence le deuxième implant 20 auto-expansif, qui est en permanence, contraint vers sa position d'équilibre.

Dans un exemple, le diamètre de l'ouverture intérieure 40, noté D, de la couronne élastique 18 au repos et le diamètre du contour externe 42, noté $D_{ext}$ sont des paramètres déterminés, avant l'implantation, à partir du diamètre du premier implant 16, noté $D_1$, et du diamètre du deuxième implant 20, noté $D_2$.

Avantageusement pour obtenir une connexion optimale entre le deuxième implant 20 et la couronne 18, il est souhaité que $D=2/3\ D_2$.

Le diamètre du contour extérieur $D_{ext}$ est défini en fonction du diamètre de l'ouverture intérieure 40 selon l'équation suivante : $D_{ext}=4/3*D*x$, où x est un facteur dépendant de la surface d'appui 44.

En outre, pour obtenir une pénétration du deuxième implant 20 dans le premier implant 10 égal à 10 % du diamètre $D_1$ du premier implant 16, il est avantageux que le diamètre $D_{ext}$ du contour extérieur 42 de la couronne 18 soit égale à 20 % du périmètre extérieur du premier implant 16.

Cela signifie qu'il est préférable que $D_{ext}=0.2\ \pi\ D_1$.

Ainsi, avec ces conditions le facteur x dépendant de la surface d'appui est déductible de l'équation suivante :

$$x=0.2\ \pi\ *\ 8/9*\ D_1/D_2.$$

Un deuxième dispositif selon l'invention (non représenté) va maintenant être décrit. A la différence du premier dispositif 10, le corps tubulaire 22 du premier implant 16 comporte un treillis formé d'au moins un fil, la couronne élastique 18 étant formée sur le corps tubulaire du premier implant dans la fenêtre à l'aide d'au moins un fil 72 du treillis. Dans ce deuxième dispositif, la couronne élastique 18 est directement tressée avec les fils 72 du premier implant 16. Dans ce dispositif, la couronne élastique 18 est venue de matière avec l'armature 30 du premier implant 18. Cela améliore la tenue du dispositif.

Dans un troisième dispositif de traitement selon l'invention, la fenêtre 26 est disposée dans l'axe A-A' du corps tubulaire 22. L'axe central B-B' de la couronne 18 est alors sensiblement colinéaire à l'axe A-A' du corps tubulaire 22. A la différence du premier dispositif 10, le premier implant 16 définit une fenêtre 26 axiale dans laquelle est inséré un deuxième implant 20.

La fenêtre 26 axiale est par exemple définie au niveau de l'ouverture distale du corps tubulaire 22. En variante, la fenêtre 26 est au niveau définie au niveau l'ouverture proximale du corps tubulaire 22.

Le premier implant 16 comporte une couronne élastique 18 s'étendant dans la fenêtre axiale. La surface d'appui 44 de la couronne 18 s'étend transversalement à l'axe A-A' au niveau de la fenêtre 26.

Le troisième dispositif sert, par exemple à jointoyer deux implants 16, 20 consécutifs. En effet, les axes A-A' du premier implant et du deuxième implant 20 sont sensiblement colinéaires. Les deux passages centraux 24, 84 communiquent sans coude.

Dans le troisième dispositif, le deuxième implant est mobile axialement dans le premier implant 16, et retenu de manière étanche par une couronne élastique 18. Cela permet à l'opérateur d'ajuster la position axiale relative des implants l'un par rapport à l'autre, pour s'adapter à des conformations anatomiques différentes.

Dans une variante, la couronne élastique 18 peut être utilisée sans deuxième implant 20, par exemple pour réduire le débit de passage de fluide dans le conduit principal 12. La couronne 18, du fait de ses propriétés élastiques, s'adapte aux mouvements imposés par le flux du liquide circulant dans le premier implant 16.

Dans l'exemple, huit brins sont représentés. Cependant, le nombre de brins peut être différent tant qu'il y a au moins trois brins 60, de préférence au moins quatre brins 60, pour définir le contour 46 de l'ouverture intérieure 40 de la couronne 18.

Le nombre de paires 66 de brins 60 est avantageusement compris entre 2 et 8.

En variante, la disposition des brins 60 est différente. Par exemple, les extrémités 62, 64 des brins 60 sur le contour externe 42 est différentes. Par exemple, plusieurs brins 60 présentent des longueurs différentes.

Dans l'exemple représenté sur la figure 1, le premier implant 16 est une endoprothèse qui comporte une couche de revêtement 32. Dans une variante, le premier implant 16 est un stent qui ne comporte pas de couche de revêtement 32 sur l'armature 30.

En variante, le corps 22 sur lequel est disposée la couronne 18 est non tubulaire.

## Revendications

1. Dispositif de traitement (10) comprenant :

    - un premier implant (16) comportant un corps (22), de préférence tubulaire, définissant une fenêtre (26) ; et
    - une couronne élastique (18), disposée dans la fenêtre (26), la couronne élastique (18) définissant une ouverture intérieure (40), un contour externe (42) et une surface d'appui (44) annulaire entre l'ouverture intérieure (40) et le contour externe (42), la couronne (18) étant déformable entre une position de repos et une position d'introduction, dans laquelle l'ouverture intérieure (40) présente un diamètre supérieur à son diamètre dans la position de repos, la couronne (18) étant sollicitée élastiquement vers la position de repos,

    **caractérisée en ce que** la couronne (18) comporte au moins trois brins (60) traversant la couronne (18), chaque brin (60) comportant une première extrémité (62) et une deuxième extrémité (64) positionnées sur le contour externe (42), le contour (46) de l'ouverture intérieure (40) étant défini par une portion (70) de chaque brin (60) et présentant une forme de pseudo-polygone.

2. Dispositif de traitement (10) selon la revendication 1, dans lequel les brins (60) sont formés à partir d'un même fil (72), de préférence tressé.

3. Dispositif de traitement (10) selon l'une quelconque des revendications 1 ou 2, dans lequel les brins (60) s'étendent sensiblement dans un même plan.

4. Dispositif de traitement (10) selon l'une quelconque des revendications précédentes, dans lequel la largeur de la surface d'appui (44) est comprise entre 30 % et 60 % du diamètre de l'ouverture intérieure (40) dans la position de repos.

5. Dispositif de traitement (10) selon l'une quelconque des revendications précédentes, comprenant au moins une paire (66) de brins (60) comprenant deux brins appariés (68), les brins appariés (68) étant des brins (60) reliés entre eux à la fois par leur première extrémité (62) et par leur deuxième extrémité (64).

6. Dispositif de traitement (10) selon la revendication 5, dans lequel les brins appariés (68) définissent une forme oblongue, notamment une forme d'œil.

7. Dispositif de traitement (10) selon l'une quelconque des revendications 5 ou 6, dans lequel les extrémités (62, 64) des paires (66) de brins (60, 68) sont réparties sur le contour externe (42).

8. Dispositif de traitement (10) selon l'une quelconque des revendications 5 à 7, comprenant un nombre pair de brins (60), les brins (60) étant appariés deux à deux, la couronne (18) comprenant de préférence huit brins (60).

9. Dispositif de traitement (10) selon l'une quelconque des revendications précédentes, dans lequel la déformation de la couronne (18) est une déformation élastique permettant une augmentation réversible d'au moins 20 %, avantageusement d'au moins 30 % du diamètre de l'ouverture intérieure (40).

10. Dispositif de traitement (10) selon l'une quelconque des revendications précédentes, dans lequel chaque brin (60) présente un diamètre compris entre 0,1 mm et 2 mm et de préférence de 0,2 mm.

11. Dispositif de traitement (10) selon l'une quelconque des revendications précédentes, dans lequel la couronne (18) est disposée sur le corps (22) du premier implant (16) dans la fenêtre (26), l'ouverture intérieure (40) délimitant le passage d'introduction d'un deuxième implant (20).

12. Dispositif de traitement (10) selon l'une quelconque des revendications précédentes, dans lequel le corps (22) est formé d'un treillis d'au moins un fil, la couronne (18) étant formée sur le corps (22) du premier implant (12) dans la fenêtre à l'aide d'au moins un fil du treillis.

13. Dispositif de traitement (10) selon l'une quelconque des revendications précédentes, dans lequel la couronne (18) est rapportée sur le corps (22) du premier implant (16) dans la fenêtre.

14. Dispositif de traitement (10) selon l'une quelconque des revendications précédentes, dans lequel la fenêtre (26) est disposée sur la paroi (28) du corps (22).

15. Dispositif de traitement (10) selon l'une quelconque des revendications précédentes, dans lequel la fenêtre (26) est disposée dans l'axe (A-A') du corps (22).

## Patentansprüche

1. Behandlungsvorrichtung (10) umfassend:

    - ein erstes Implantat (16), das einen Körper (22) enthält, vorzugsweise rohrförmig, der eine Öffnung (26) definiert; und
    - einen elastischen Kranz (18), angeordnet in der Öffnung (26), der elastische Kranz (18) definiert eine innere Öffnung (40), einen Außenumfang (42) und eine ringförmige Auflagefläche (44) zwischen der inneren Öffnung (40) und dem Außenumfang (42), der Kranz (18) ist dabei verformbar zwischen einer Ruheposition und einer Einführungsposition, in der die innere Öffnung (40) einen größeren Durchmesser aufweist als der Durchmesser in der Ruheposition, der Kranz (18) wird dabei elastisch zur Ruheposition hin beansprucht,

**dadurch gekennzeichnet, dass** der Kranz (18) mindestens drei Fasern (60) enthält, die durch den Kranz (18) hindurchführen, jede Faser (60) enthält dabei ein erstes Endstück (62) und ein zweites Endstück (64), angeordnet auf dem Außenumfang (42), der Umfang (46) der inneren Öffnung (40) ist dabei definiert durch einen Abschnitt (70) jeder Faser (60) und weist die Form eines Pseudopolygons auf

2. Behandlungsvorrichtung (10) nach Anspruch 1, bei der die Fasern (60) ausgehend vom selben Faden (72) gebildet werden, vorzugsweise geflochten.

3. Behandlungsvorrichtung (10) nach einem beliebigen der Ansprüche 1 oder 2, bei dem die Fasern (60) sich im Wesentlichen in derselben Ebene erstrecken.

4. Behandlungsvorrichtung (10) nach irgendeinem der vorangehenden Ansprüche, bei dem die Breite der Auflagefläche (44) bei zwischen 30 % und 60 % des Durchmessers der inneren Öffnung (40) in der Ruheposition liegt.

5. Behandlungsvorrichtung (10) nach einem beliebigen der vorangehenden Ansprüche, umfassend mindestens ein Paar (66) Fasern (60), das zwei verpaarte Fasern (68) enthält, bei den verpaarten Fasern (68) handelt es sich um Fasern (60), die miteinander sowohl über ihr erstes Endstück (62) wie über ihr zweites Endstück (64) verbunden sind.

6. Behandlungsvorrichtung (10) nach Anspruch 5, bei dem die gepaarten Fasern (68) eine längliche Form definieren, insbesondere eine Augenform.

7. Behandlungsvorrichtung (10) nach einem beliebigen der Ansprüche 5 oder 6, bei dem die Endstücke (62,64) der Paare (66) der Fasern (60, 68) über den Außenumfang (42) verteilt sind.

8. Behandlungsvorrichtung (10) nach einem beliebigen der Ansprüche 5 bis 7, umfassend eine gerade Anzahl Fasern (60), die Fasern (60) sind jeweils zu zweit gepaart, der Kranz (18) umfasst vorzugsweise acht Fasern (60).

9. Behandlungsvorrichtung (10) nach einem beliebigen der vorangehenden Ansprüche, bei denen die Verformung des Kranzes (18) eine elastische Verformung ist, die eine reversible Erhöhung von mindestens 20 % erlaubt, vorteilhafterweise mindestens 30 % des Durchmessers der inneren Öffnung (40).

10. Behandlungsvorrichtung (10) nach einem beliebigen der vorangehenden Ansprüche, bei der jede Faser (60) einen Durchmesser zwischen 0,1 mm und 2 mm aufweist und vorzugsweise von 0,2 mm.

11. Behandlungsvorrichtung (10) nach einem beliebigen der vorangehenden Ansprüche, bei der der Kranz (18) auf dem Körper (22) des ersten Implantats (16) in der Öffnung (26) angeordnet ist, die innere Öffnung (40) begrenzt dabei die Einführungspassage eines zweiten Implantats (20).

12. Behandlungsvorrichtung (10) nach einem beliebigen der vorangehenden Ansprüche, bei der der Körper (22) aus einem Geflecht mit mindestens einem Faden geformt ist, der Kranz (18) wird dabei auf dem Körper (22) des ersten Implantats (12) in der Öffnung mit Hilfe mindestens eines Gewebefadens gebildet.

13. Behandlungsvorrichtung (10) nach einem beliebigen der vorangehenden Ansprüche, bei der der Kranz (18) auf dem Körper (22) des ersten Implantats (16) in der Öffnung versetzt ist.

14. Behandlungsvorrichtung (10) nach einem beliebigen der vorangehenden Ansprüche, bei der der die Öffnung (26) auf der Wand (28) des Körpers (22) angeordnet ist.

15. Behandlungsvorrichtung (10) nach einem beliebigen der vorangehenden Ansprüche, bei der die Öffnung (26) in der Achse (A-A') des Körpers (22) angeordnet ist.

## Claims

1. Treatment device (10) comprising:

- a first implant (16) comprising a body (22), preferably tubular, defining a window (26); and
- an elastic ring (18), arranged in the window (26), the elastic ring (18) defining an inner opening (40), an outer contour (42) and an annular bearing surface (44) between the inner opening (40) and the outer contour (42), the ring (18) being deformable between a rest position and an introduction position, in which the inner opening (40) has a diameter greater than its diameter in the rest position, the ring (18) being elastically biased towards the rest position,
**characterized in that** the ring (18) comprises at least three strands (60) passing through the ring (18), each strand (60) comprising a first end (62) and a second end (64) positioned on the outer contour (42), the contour (62) of the opening (40) being defined by a portion (70) of each strand (60) and having a pseudo-polygon shape.

2. Treatment device (10) according to claim 1, wherein the strands (60) are formed from the same yarn (72), preferably braided.

3. Treatment device (10) of any one of claims 1 or 2, wherein the strands (60) extend substantially in the same plane.

4. Treatment device (10) according to any one of the preceding claims, wherein the width of the bearing surface (44) is between 30% and 60% of the diameter of the inner opening (40) in the rest position.

5. Treatment device (10) according to any one of the preceding claims, comprising at least one pair (66) of strands (60) comprising two paired strands (68), the paired strands (68) being strands (60) connected to one another both by their first end (62) and by their second end (64).

6. Treatment device (10) according to claim 5, wherein the paired strands (68) define an oblong shape, including an eye shape.

7. Treatment device (10) according to any one of claims 5 or 6, wherein the ends (62, 64) of the pairs (66) of strands (60, 68) are distributed over the outer contour (42).

8. Treatment device (10) according to any one of Claims 5 to 7, comprising an even number of strands (60), the strands (60) being paired in pairs, the ring (18) preferably comprising eight strands (60).

9. Treatment device (10) according to any one of the preceding claims, wherein the deformation of the ring (18) is an elastic deformation allowing a reversible increase of at least 20%, advantageously at least 30% of the diameter of the inner opening (40).

10. Treatment device (10) according to any one of the preceding claims, wherein each strand (60) has a diameter comprised between 0.1 mm and 2 mm, and preferably 0.2 mm.

11. Treatment device (10) according to any one of the preceding claims, wherein the ring (18) is disposed on the body (22) of the first implant (16) in the window (26), the inner opening (40) defining the introduction passage of a second implant (20).

12. Treatment device (10) according to any one of the preceding claims, wherein the body (22) is formed of a mesh of at least one wire, the ring (18) being formed on the body (22) of the first implant (12) in the window using at least one wire of the mesh.

13. Treatment device (10) according to any one of the preceding claims, wherein the ring (18) is fitted to the body (22) of the first implant (16) in the window.

14. Treatment device (10) according to any one of the preceding claims, wherein the window (26) is disposed on the wall (28) of the body (22).

15. Treatment device (10) according to any one of the preceding claims, wherein the window (26) is arranged in the axis (A-A') of the body (22).

**FIG.2**

**FIG.1**

FIG.3

FIG.4

FIG.5

FIG.6

## RÉFÉRENCES CITÉES DANS LA DESCRIPTION

**Documents brevets cités dans la description**

- EP 2895110 A
- WO 2005034810 A
- EP 2749252 A1
- US 20030120292 A1